# EUROPEAN PATENT SPECIFICATION

(11) **EP 3 606 438 B1**
(45) Date of publication and mention of the grant of the patent: **14.10.2020**
(21) Application number: 18717262.2
(22) Date of filing: 29.03.2018
(51) Int. Cl.: A61B 8/08, A61B 8/00, A61B 5/20, A61B 5/107

(54) **BLADDER MONITORING**
BLASENÜBERWACHUNG
CONTRÔLE DE LA VESSIE

(30) Priority: 03.04.2017 EP 17164540
(43) Date of publication of application: 12.02.2020
(73) Proprietor: Koninklijke Philips N.V., 5656 AG Eindhoven (NL)
(72) Inventor: VAN HEESCH, Christianus Martinus, 5656 AE Eindhoven (NL); PAULUSSEN, Igor Wilhelmus Franciscus, 5656 AE Eindhoven (NL); DE WILD, Nico Maris Adriaan, 5656 AE Eindhoven (NL); DONATO, Katia, 5656 AE Eindhoven (NL); BINGLEY, Peter, 5656 AE Eindhoven (NL); WANG, Ke, 5656 AE Eindhoven (NL); VAN HEESCH, Franciscus Hendrikus, 5656 AE Eindhoven (NL); MERT, Aydin, 5656 AE Eindhoven (NL); BEZEMER, Rick, 5656 AE Eindhoven (NL); VAN RENS, Antonia Cornelia, 5656 AE Eindhoven (NL); BUDZELAAR, Franciscus Paulus Maria, 5656 AE Eindhoven (NL)
(74) Representative: Philips Intellectual Property & Standards
(86) International application number: PCT/EP2018/058032
(87) International publication number: WO 2018/184962

(56) References cited:
- WO-A2-02/094089
- WO-A2-2005/034717
- US-A1- 2011 004 123
- US-B1- 6 406 431

## Description

### FIELD OF THE INVENTION

The present invention relates to a bladder monitoring system comprising a wearable bladder monitoring device and to a method of monitoring a bladder with such a system.

### BACKGROUND OF THE INVENTION

In medicine, there is an on-going interest in monitoring the bladder volume of a subject, e.g. a patient. As is well-known per se, the bladder is a triangularly shaped hollow organ for storing urine, with elastic walls such that the bladder can expand upon receiving urine from the kidneys before expelling the urine through the subject's urethra.

There are a number of reasons why such bladder volume monitoring is of interest. For example, the bladder retention volume (i.e. the amount of urine retained by the bladder after the subject's urinating) is an indicator of prostate and urinary tract conditions, which conditions may be triggered by bacteria and other pathogens in the retained urine; consequent bladder stretching is a key contributor to the occurrence of delirium in elderly subjects; the bladder filling rate is an indicator for the hydration state and kidney function of the subject, and so on. In addition to such diagnostic reasons, bladder volume monitoring may be used as a tool for bladder control training, as well as used as a monitor of a hydration state of the monitored subject.

It is desirable to have a wearable bladder monitor to facilitate (semi-)continuous bladder monitoring. An example of such a wearable bladder monitor is disclosed in US 2007/0167768 A1, which discloses a non-invasive method and apparatus for monitoring the bladder volume in humans or animals. The monitoring measurements are based on the analysis of ultrasound signals from at least two individual ultrasound transducer arrays. Each one individual ultrasound transducer array has a contact face, which is arranged in order to transmit an ultrasonic signal through an abdominal surface of an individual being monitored. The method and apparatus includes the arranging of each individual transducer array on the abdominal surface around an axis which extends from a point on the abdominal surface through a point on or within the urinary bladder. The method and apparatus further comprises that each individual transducer array is arranged to scan the bladder using a two-dimensional scan plane which extends radially from the transducer array through the bladder.

In practice, this apparatus suffers from a number of drawbacks. Firstly, it is rather involved in terms of signal processing, which therefore reduces the battery life of the apparatus, even if the bulk of the signal processing is done remotely. What is more, the operation of the apparatus is based on a presumed fixed relationship between the orientation of the ultrasound transducer arrays of the apparatus and the bladder of the individual. In practice, this fixed relationship does not exist such that the bladder volume estimations provided with this apparatus may be unreliable in certain scenarios, e.g. depending on the body posture of the individual, e.g. the individual standing, lying down, sitting, crouching, and so on. Also, in order to estimate the bladder volume, a full (unimpeded) view of the bladder by the ultrasound transducer arrays may be required. Such an unimpeded view is typically not available, at least not in adult individuals, as in such individuals the bladder is at least partially obscured by the pelvic bone.

WO 02/094089 A2 discloses a bladder monitoring system comprising:
1. a wearable bladder monitoring device including:
   - securing means for securing the device to a subject's body;
   - a phased array of ultrasound transducers; and
   - a phased array controller adapted to control the phased array to direct a plurality of ultrasound beams into the subject's body under a range of beam angles; and
2. a signal processor adapted to receive data pertaining to echo signals of said ultrasound beams from the phased array and to process said data in order to derive bladder information from the data based on the trajectories of the ultrasound beams passing through the bladder.

This system forms the closest prior art for the invention.

### SUMMARY OF THE INVENTION

The present invention seeks to provide a non-invasive bladder monitoring system that does not need to rely on actual bladder volume estimation for bladder monitoring purposes.

The present invention further seeks to provide a non-invasive method of monitoring a bladder with such a bladder monitoring system.

According to an aspect, there is provided a bladder monitoring system comprising a wearable bladder monitoring device including securing means for securing the device to a subject's body; a phased array of ultrasound transducers; and a phased array controller adapted to control the phased array to direct a plurality of ultrasound beams into the subject's body under a range of beam angles; and a signal processor adapted to receive data pertaining to echo signals of said ultrasound beams from the phased array and to process said data in order to identify an edge of the subject's pelvic bone proximal to the subject's bladder from data pertaining to at least one of said echo signals; determine an orientation of the wearable bladder monitoring device relative to the pelvic bone based on beam angle information associated with the at least one of said echo signals; and derive bladder information from the data based on the trajectories of the ultrasound beams passing through the bladder as a function of the determined orientation of the wearable bladder monitoring device relative to the pelvic bone.

The present invention is based on the insight that by generating a plurality of ultrasound beams under a range of beam angles with such a phased array, the echoes of such ultrasound beams can be categorized into three distinguishable categories, namely a first category of echoes incident on the pelvic bone, a second category of echoes passing through the bladder and a third category of echoes passing through tissue adjacent to the bladder. In this manner, based on the beam angle information the orientation of the wearable bladder monitoring device relative to the pelvic bone can be retrieved from the echoes, which orientation can be used to accurately estimate the bladder volume from the subset of echoes relating to ultrasound beams passing through the bladder, which echoes may be further sub-categorized in echoes passing through the bladder wall and echoes passing through urine in the bladder.

In some embodiments of the present invention, the bladder monitoring device does not necessarily form part of the bladder monitoring system, as the data processing of such a bladder monitoring device may be performed on a separate device comprising the signal processor, e.g. a remote (electronic) device forming the bladder monitoring system. In order to maintain battery life, the processing of the echo signals acquired with the phased array may be performed on a remote device containing the signal processor, e.g. a wearable smart device such as a smart watch, a portable smart device such as a mobile phone or tablet computer, a laptop computer, a personal computer or the like, as such signal processing typically is rather computationally intensive. To this end, the wearable bladder monitoring device may be configured to perform some pre-processing of the echo signals, e.g. down conversion or the like, in order to reduce the amount of data that needs to be communicated to the remote device, thereby further extending battery life. However, in an alternative embodiment the wearable bladder monitoring device further comprises the signal processor, thereby providing a self-contained device, which may be configured to communicate minimal amounts of data, e.g. processing results, to a remote device, e.g. for visualization purposes.

In an embodiment, the wearable bladder monitoring device further comprises an orientation sensor for determining an orientation of the subject, and wherein the signal processor is adapted to determine an orientation of the wearable bladder monitoring device relative to the pelvic bone based on beam angle information associated with the at least one of said echo signals and an orientation signal from the orientation sensor. Such an orientation sensor, e.g. an accelerometer or the like, may be used to help determine the body posture of the subject, to further aid the accuracy of any bladder data processing, e.g. bladder volume estimation.

For example, the wearable bladder monitoring device may further comprise a data storage device storing a plurality of bladder models, each associated with a particular orientation of the subject, wherein the signal processor is adapted to retrieve the defined bladder model from the data storage device based on the received orientation signal. In this manner, a bladder model that is particularly accurate for a particular body posture of the subject may be deployed, e.g. in response to the information provided with the orientation sensor, to further improve the accuracy of the processing of the bladder monitoring data acquired with the phased array.

In an embodiment, the signal processor is adapted to estimate a diameter of the subject's bladder for each echo signal from the data pertaining to a subset of said echo signals. In the context of the present application, the term 'diameter' is used to refer to a cross-sectional length or width of the bladder between opposing bladder wall sections through which the ultrasound beam responsible for the echo signal propagates. Such a collection of diameters may be used to estimate a volume of the bladder, e.g. using an appropriate bladder model, and to derive a fill level of the bladder from the estimated volume. For example, the signal processor may be adapted to estimate a bladder volume by fitting the estimated diameters of the subject's bladder to a defined bladder model and estimating the bladder volume from the fitting result.

In a particularly advantageous embodiment, the signal processor is further adapted to, for each echo signal in said subset, calculate a first optimal frequency of its associated ultrasound beam for imaging an anterior boundary of the bladder; calculate a second optimal frequency of its associated ultrasound beam for imaging a posterior boundary of the bladder; instruct the phased array controller to generate at least one further ultrasound beam under the beam angle of the associated ultrasound beam with the phased array, said at least at least one further ultrasound beam having a frequency corresponding to at least one of the first optimal frequency and the second optimal frequency or an average of the first optimal frequency and the second optimal frequency; and estimate the diameter of the subject's bladder for said beam angle from the at least one further echo signal of the at least one further ultrasound beam. This facilitates a more accurate determination of the bladder diameter as the opposing boundaries (wall portions) of the bladder are more accurately imaged, thereby improving the accuracy of the interpretation of the bladder data acquired with a phased array, e.g. an estimated bladder volume and/or fill level. The at least one further ultrasound beam may comprise a first further ultrasound beam having the first optimal frequency and a second further ultrasound beam having the second optimal frequency.

The phased array controller may be adapted to periodically generate said plurality of ultrasound beams in order to monitor changes to a bladder state over time. In order to optimise the battery life of the wearable bladder monitoring device, a frequency of said periodic generation is based on a previously determined bladder function by the signal processor. The signal processor may be adapted to derive a bladder function from the data pertaining to the respective echo signals of the periodically generated pluralities of ultrasound beams, e.g. to monitor a change in a fill level of the bladder over time, from which useful information such as the subject's dehydration or rehydration rates may be derived.

The securing means may include a strap, e.g. a belt, attached to the wearable bladder monitoring device and/or an adhesive layer on a subject-facing surface of the wearable bladder monitoring device. The adhesive layer is particularly preferred as this is capable of securely fastening the wearable bladder monitoring device with minimal risk of the device accidentally moving into another location relative to the subject's bladder, and has the further advantage of being minimally intrusive compared to the strap, which may be perceived as less comfortable at least by some users.

In order to facilitate communication between the wearable bladder monitoring device and a remote device as previously explained, the wearable bladder monitoring device may further comprise a wireless communication module for communicating a processing result of the signal processor to a remote device or to communicate (pre-processed) echo signals to a signal processor of such a remote device.

Preferably, the ultrasound transducers are capacitive micromachined ultrasound transducers (CMUTs) or piezoelectric micromachined ultrasound transducers (PMUTs), or piezoelectric ceramic transducers, with CMUTs being particularly preferred due to the large resonance frequency range of such transducers, which makes them particularly suitable for use in the wearable bladder monitoring device according to embodiments of the present invention.

According to another aspect, there is provided a method of monitoring a bladder with the bladder monitoring system of any of the herein described embodiments, the method comprising securing the wearable bladder monitoring device to a subject's body; generating a plurality of ultrasound beams under a range of beam angles with the phased array; receiving echo signals of said ultrasound beams from the phased array; and processing data pertaining to said echo signals in order to identify an edge of the subject's pelvic bone proximal to the subject's bladder from data pertaining to at least one of said echo signals; determine an orientation of the wearable bladder monitoring device relative to the pelvic bone based on beam angle information associated with the at least one of said echo signals; and derive bladder information from the data pertaining based on the trajectories of the ultrasound beams passing through the bladder as a function of the determined orientation of the wearable bladder monitoring device relative to the pelvic bone.

### BRIEF DESCRIPTION OF THE DRAWINGS

Embodiments of the invention are described in more detail and by way of non-limiting examples with reference to the accompanying drawings, wherein:
FIG. 1 schematically depicts a bladder monitoring system according to an embodiment;
FIG. 2 schematically depicts an operating principle of a bladder monitoring system according to an embodiment;
FIG. 3 schematically depicts an aspect of a bladder monitoring system according to an embodiment;
FIG. 4 schematically depicts an aspect of a bladder monitoring system according to another embodiment;
FIG. 5 schematically depicts a bladder monitoring system according to an embodiment in use on a subject;
FIG. 6 schematically depicts an aspect of typical echo signals received by a bladder monitoring system according to an embodiment;
FIG. 7 schematically depicts an angular dependence of ultrasound signals passing through a subject's bladder resulting from orientations of the bladder monitoring system according to an embodiment relative to the subject;
FIG. 8 is a flowchart of an example embodiment of a method of monitoring a bladder with a bladder monitoring system according to an embodiment;
FIG. 9 schematically depicts a bladder monitoring system according to another embodiment; and
FIG. 10 is a flowchart of an aspect of another example embodiment of a method of monitoring a bladder with a bladder monitoring system according to an embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

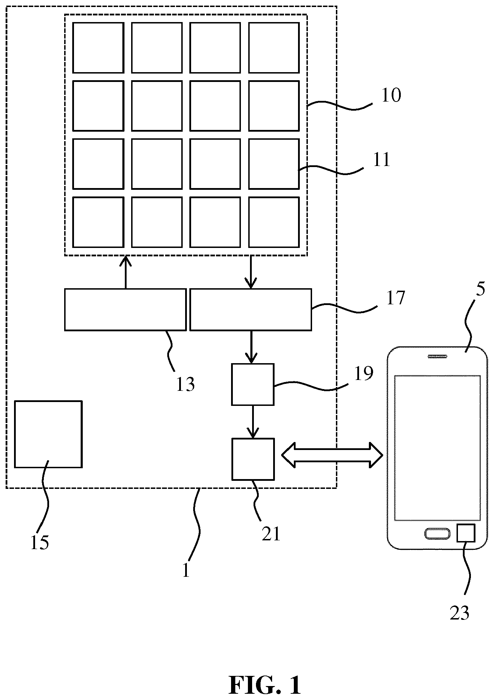

FIG. 1 schematically depicts a bladder monitoring system according to an example embodiment. The bladder monitoring system comprises a wearable bladder monitoring device 1 that may be secured to an individual (subject) using suitable securing means, such as a strap or belt, or an adhesive layer as will be explained in more detail later. The wearable bladder monitoring device 1 comprises a phased array 10 of ultrasound transducer elements 11 under control of a phased array controller 13. Any suitable type of ultrasound transducer elements 11 may be used for this purpose, e.g. PZT elements, CMUT elements, PMUT elements, and so on, although CMUT elements are particularly preferred, in particular over PZT elements due to their superior (adjustable) resonance frequency range, which make CMUT elements particularly suitable for bladder monitoring purposes. As such transducer elements are well-known per se, they will not be explained in further detail for the sake of brevity only.

The phased array 10 may take any suitable shape, e.g. a one-dimensional array or two-dimensional array of ultrasound transducer elements 11 of any suitable size, such as a 4x4 mm array 10 having 16 ultrasound transducer elements 11 with a pitch in a range of 200-300 microns, by way of non-limiting example. Other dimensions are of course equally feasible. In an embodiment, the ultrasound transducer elements 11 are operable in a frequency range of 2-12 MHz, although other frequency ranges may be contemplated. As will be explained in more detail below, preferably the operation frequency of the ultrasound transducer elements 11 may be altered, although in alternative embodiment the ultrasound transducer elements 11 are arranged to be operated at a fixed resonance frequency, e.g. about 3-4 MHz.

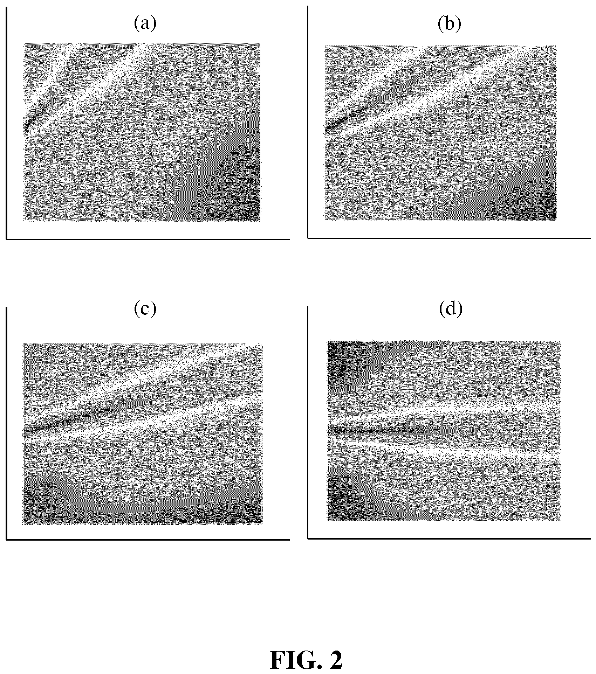

The phased array controller 13 typically is arranged to steer ultrasound beams generated with the ultrasound transducer elements 11 of the phased array 10 under a range of angles, as schematically depicted in FIG. 2, in which by varying the delay times between the firing of the individual (lines of) ultrasound transducer elements 11 as is well-known per se, beam angles may be generated under a number of beam angles, e.g. 45° (a), 30° (b), 15° (c) and 0° (d), wherein the beam angle is defined relative to the transmission surface of the phased array 10. In an embodiment, the beam angle range ranges from 0° to 45°, in which beam angles are altered in 15° increments, although a larger or smaller beam angle range utilizing larger or smaller beam angle increments of course may also be contemplated.

Now, upon returning to FIG. 1, the phased array 10 typically is operable in a transmit mode in which the ultrasound beams are generated under control of the phased array controller 13 and a receive mode in which the phased array is operable to receive echo signals induced by the generated ultrasound beams within the body of the individual wearing the wearable bladder monitoring device 1. As will be readily understood by the skilled person, the wearable bladder monitoring device 1 may be operated such that a transmit mode of an ultrasound beam under a particular beam angle is followed by the receive mode of its induced echo signals, before the next transmit mode of an ultrasound beam under a further particular beam angle is initiated, such as to ensure that for each ultrasound beam its echoes do not suffer interference from echoes of ultrasound beams under different beam angles.

The echo signals may be pre-processed by a down conversion stage 17, which may form part of the phased array controller 13 or may be a separate entity. The down conversion stage 17 may apply noise filtering and frequency down conversion of the echo signals, e.g. from the MHz domain to the KHz domain, and employ a signal envelope to the down converted echo signals to reduce the amount of data that needs to be provided to a signal processor for deriving the desired bladder information from the acquired echo signals. Other suitable data reduction techniques will be immediately apparent to the skilled person.

In FIG. 1, the signal processor 23 forms part of a remote device 5, e.g. a wearable smart device such as a smart watch, a portable smart device such as a mobile phone or tablet computer, a laptop computer, a personal computer, or the like in order to reduce the computational effort required from the wearable bladder monitoring device 1 such that the lifetime of the battery 15 of the wearable bladder monitoring device 1 may be extended. The remote device 5 may be configured to obtain the desired functionality for the signal processor 23 in any suitable manner, e.g. by means of a software program installed onto the remote device 5, such as an app or the like.

To this end, the down conversion stage 17 may be communicatively coupled to a data storage device 19, e.g. a memory or the like, on-board the wearable bladder monitoring device 1, with a wireless communication module 21 communicatively coupled to the data storage device 19 such that the data pertaining to the acquired echo signals as stored in the data storage device 19 may be periodically communicated to the remote device 5, e.g. in response to a communication request from the remote device. Any suitable wireless communication protocol, e.g. Bluetooth, Wi-Fi, a mobile communication protocol such as 3G, 4G, 5G or the like, a near field communication protocol (NFC), and so on, may be deployed for the communication between the wearable bladder monitoring device 1 and the remote device 5. As mentioned previously, the data pertaining to the acquired echo signals communicated from the wearable bladder monitoring device 1 to the remote device 5 preferably is reduced in size to minimize the amount of data that needs to be communicated in this manner in order to extend the lifetime of the battery 15. However, it should be understood that in alternative embodiments the signal processor 23 may be contained within the wearable bladder monitoring device 1. In such embodiments, the wireless communication module 21 may be used to communicate a processing result of the signal processor 23 to the remote device 5, e.g. for visualization of the processing result on a display 7 of the remote device 5.

In yet a further embodiment, the wearable bladder monitoring device 1 is configured to upload the data pertaining to the acquired echo signals with a wireless communication module 21 into a remote data repository acting as an intermediary data storage device from which the remote device 5 may download the stored data. This has the advantage that the remote device 5 does not have to be within communication range of the wearable bladder monitoring device 1 but instead may download the data at any suitable point in time to evaluate this data. For example, the remote data repository may be a cloud storage solution or the like, which may be accessed by both the wearable bladder monitoring device 1 and the remote device 5 over a network connection such as the Internet, in which case the wearable bladder monitoring device 1 may establish a wireless connection with an Internet hub such as a router or the like within communication range of the wireless communication module 21 through which the data pertaining to the acquired echo signals may be uploaded into the remote data repository.

Any suitable type of battery 15 may be used within the wearable bladder monitoring device 1. The battery 15 may be non-rechargeable, which for example may be acceptable where the wearable bladder monitoring device 1 is a disposable device. Alternatively, the battery 15 may be a rechargeable battery in which case the wearable bladder monitoring device 1 may comprise a recharging port (not shown) through which the battery 15 may be recharged in any suitable manner. In the absence of such a recharging port, the battery 15 may be recharged using wireless recharging, e.g. NFC recharging as is well-known per se.

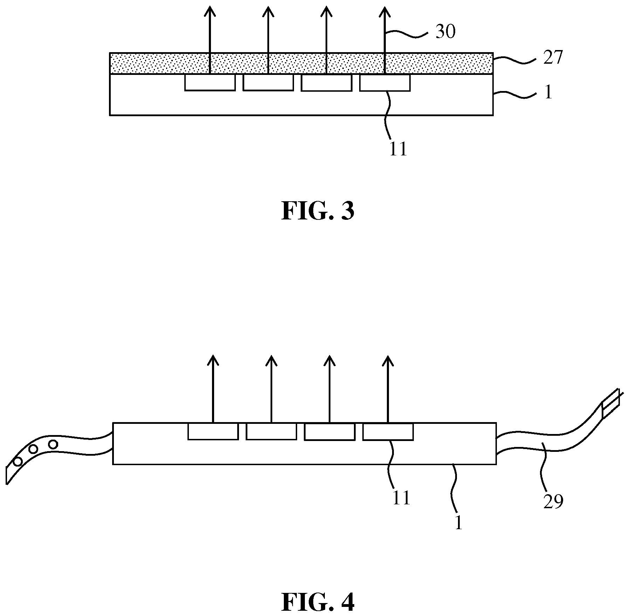

FIG. 3 schematically depicts a cross-section of a wearable bladder monitoring device 1 in which a subject-facing surface of the wearable bladder monitoring device 1 is covered with an adhesive layer 27, e.g. an adhesive polymer layer or the like, for adhering the wearable bladder monitoring device 1 to an abdominal skin region of the subject, such that ultrasound beams 30 originating from the ultrasound transducer elements 11 can be steered into the subject's body under control of the phased array controller 13 as previously explained. The use of an adhesive layer 27 has the advantage of achieving a particularly secure fit between the abdominal skin region of the subject and the wearable bladder monitoring device 1, in particular if no hair is present in this skin region.

FIG. 4 schematically depicts a cross-section of a wearable bladder monitoring device 1 in which the wearable bladder monitoring device 1 comprises a strap 29 for securing the bladder monitoring device 1 against the abdominal skin region of the subject. Such a strap 29 may be secured against the subject in any suitable manner, e.g. using a fastening member arrangement such as a belt buckle at one end of the strap 29 and holes for receiving the belt buckle at another end of the strap 29, a fastening clip through which the strap 29 can be adjusted in an non-engaged configuration of the clip and in which the strap 29 can be affixed in an engaged configuration of the clip, a Velcro arrangement, and so on. It should furthermore be understood that the strap 29 does not necessarily include a fastening member arrangement; alternatively, the strap 29 may be a closed loop, e.g. a closed belt or the like, which is elastic such that the strap 29 can be stretched to position the wearable bladder monitoring device 1 over the desired abdominal skin region of the subject, such that the elastic force of the stretched elastic strap 29 retains the wearable bladder monitoring device 1 in the desired position and preferably in a preferred orientation. In further embodiments, the wearable bladder monitoring device 1 may have a fastening arrangement in which the adhesive layer 27 is combined with a strap 29 according to any of the aforementioned embodiments.

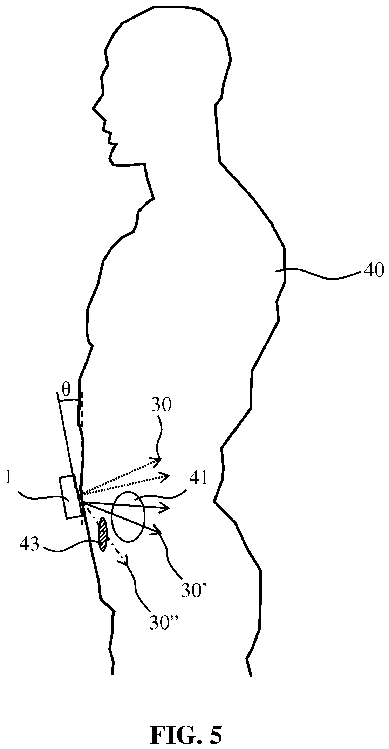

FIG. 5 schematically depicts the positioning of the wearable bladder monitoring device 1 on the abdominal region of the subject 40. The wearable bladder monitoring device 1 preferably is positioned in an abdominal region in between the pelvic bone 43 and the bellybutton (not shown) of the subject 40, e.g. a few centimetres below the belly button in order to facilitate a good viewing angle of the bladder 41 of the subject 40, i.e. to minimize the area of the bladder 41 that is obscured from direct view of a phased array 10 of ultrasound transducer elements 11. In the context of the present application, such a direct view is a transmission path of an ultrasound beam 30 towards the bladder 41 that does not first have to pass through a skeletal structure such as a bone.

As is schematically depicted in FIG. 5, the beam steering applied by the phased array controller 30 typically results in a plurality of ultrasound beams under a range of beam angles being created, wherein different (subsets of) ultrasound beams travel through different anatomical structures of the subject 40. For example, ultrasound beams 30 as indicated by the finely dashed arrows travel through tissue only, whereas ultrasound beams 30' as indicated by the solid arrows travel through a small layer of tissue before travelling through the bladder 41, whilst ultrasound beams 30" as indicated by the coarsely dashed arrow travelling towards the pelvic bone 43.

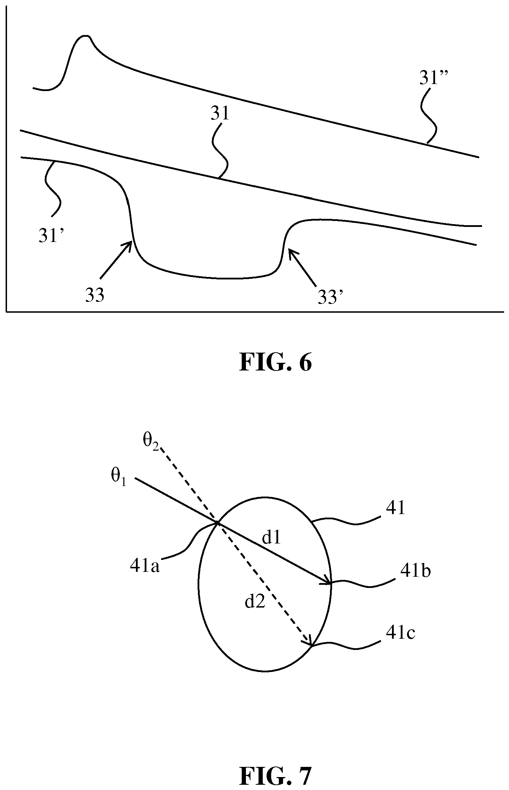

An important aspect of embodiments of the present invention is that such different paths of the ultrasound beams 30, 30', 30" lead to clearly distinguishable echo signals, as is schematically depicted in FIG. 6, in which echo signal intensities (y-axis) as a function of time (i.e. object distance from the phased array causing the signal reflection; x-axis) are depicted. Specifically, the ultrasound beams 30 travelling through tissue only typically yield an echo signal 31 having a continually decreasing signal intensity with increasing echo acquisition time delay, whereas the ultrasound beams 30' travelling through the bladder 41 cause echo signals 31' that are characterised by a sudden change 33' in the echo signal intensity, or by a pair of such signal intensity changes 33 and 33', which signal intensity changes are characteristic of the echo signals encountering the anterior wall of the bladder 41 at a first distance from the phased array 10 (signal intensity change 33) and the posterior wall of the bladder 41 at a second distance from the phased array 10 (signal intensity change 33'), with the echo signal in between these respective signal intensity changes indicative of the medium, e.g. urine or tissue, in the bladder 41 in between the anterior and posterior wall portions imaged by the ultrasound beam from which the echo signal 31' has originated. Finally, the echo signal 31" resulting from ultrasound beams 30" incident on the pelvic bone 43 is characterised by a sharp intensity peak caused by the reflection of the ultrasound beams by the pelvic bone 43 at a relatively close distance from the phased array 10.

The distance between the intensity changes in an echo signal 31' indicative of the anterior and posterior wall portions imaged by an ultrasound beam 30' from which this echo signal originates may be used to estimate a diameter of a section of the bladder 41, as schematically depicted in FIG. 7, in which an ultrasound beam 30' enters the bladder 41 through anterior wall portion 41a. However, depending on the relative orientation of the wearable bladder monitoring device 1 to the abdominal region of the subject 40, as expressed by the angle θ in FIG. 5, the ultrasound beam 30' may exit the bladder through its posterior wall portion 41b if the wearable bladder monitoring device 1 is oriented relative to the abdominal region of the subject 40 under a first angle θ₁, giving rise to an estimated diameter d1 as being the distance (time delay) between the midpoints of the edges 33 and 33' of the echo signal, which midpoints may be interpreted as the estimated locations of the anterior wall portion 41a and the posterior wall portion 41b.

However, the ultrasound beam 30' may exit the bladder through its posterior wall portion 41c if the wearable bladder monitoring device 1 is oriented relative to the abdominal region of the subject 40 under a second angle θ₂, giving rise to an estimated bladder diameter d2, with typically d2 ≠ d1. In this context, the angle θ may be defined as the angle between the subject-facing surface plane of the wearable bladder monitoring device 1 and the plane of the abdominal skin contact region onto which the wearable bladder monitoring device 1 is placed. This therefore shows that the relative orientation of the wearable bladder monitoring device 1 to the abdominal region of the subject 40 has an impact on the bladder monitoring data, in particular on the length of a path of an ultrasound beam 30' through a section of the bladder 41 as explained above.

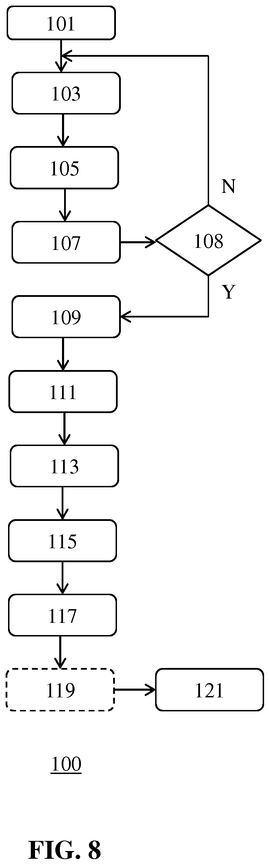

The operation of the bladder monitoring system according to embodiments of the present invention is designed to factor in such orientational dependency of the wearable bladder monitoring device 1 on the bladder monitoring results, as will now be explained in more detail with the aid of FIG. 8, in which a flowchart of a method 100 of monitoring the bladder 41 of a subject 40 with such a bladder monitoring system is shown. The method 100 starts in operation 101 with the placement of the wearable bladder monitoring device 1 in a defined location on the abdomen of the subject 40, e.g. just under the subject's bellybutton as previously explained, after which in operation 103 the phased array controller 13 controls the phased array 10 to generate a plurality of ultrasound beams 30 into the subject's body under a range of beam angles, e.g. a range from 0-45° with defined beam angle increments, e.g. 5° or 15° increments. For each generated ultrasound beam 30, an echo signal 40 is received in operation 105 before the ultrasound beam 30 under the next beam angle is generated in a repeat of operation 103. Any suitable acquisition period for the echo signals 40 may be deployed in operation 105, e.g. an acquisition period of several milliseconds.

Each received echo signal may be pre-processed by the pre-processing state 17 in operation 107, which pre-processing may include one or more or noise filtering, signal down conversion, signal enveloping and signal digitization, and which may further include storing the pre-processed signal in data storage device 19 for subsequent processing by the signal processor 23, either on a remote device 5 or on the wearable bladder monitoring device 1 as previously explained. It is checked in operation 108 if all ultrasound signals 30 under the desired beam angles have been generated, the respective echo signals 40 have been collected and pre-processed. If this is not the case, the method 100 reverts back to operation 103 in which the next ultrasound beam 30 is generated, e.g. under an adjusted beam angle.

Otherwise, the method 100 proceeds to operation 109 in which the acquired (pre-processed) ultrasound echo signals 40 are passed on to the signal processor 23, which may involve a wireless communication between the wearable bladder monitoring device 1 and the remote device 5 as previously explained in case the signal processor 23 is located on the remote device 5, in which the signal processor 23 processes the received data in order to determine the orientation angle θ of the wearable bladder monitoring device 1 relative to the abdomen of the subject 40. Specifically, the signal processor 23 evaluates the respective echo signals 40 to identify the subset of echo signals 40 reflected by the pelvic bone 43, which echo signals may be identified as previously explained with the aid of FIG. 6, in order to identify the edge of the pelvic bone 43 proximal to the bladder 41 from this subset of echo signals 40. This echo signal for example may be identified by systematically evaluating the echo signals as a function of reducing beam angle of the ultrasound beams corresponding to these echo signals in order to find the ultrasound beam 30 with the lowest beam angle leading to an echo signal reflecting off the pelvic bone 43. Because the wearable bladder monitoring device 1 is positioned in an (approximately) fixed position relative to the pelvic bone 43, the relative orientation angle θ of the wearable bladder monitoring device 1 can be derived from this beam angle information by the signal processor 23 in operation 111 of the method 100.

In operation 113, the signal processor 23 fits the echo signals 31' of the ultrasound beams 30' passing through a section of the bladder 41 to a bladder model. In operation 115, the signal processor 23 may estimate a diameter or cross-sectional length of the bladder section from the distance or time delay between the respective edges 33 and 33' of each echo signal 31' indicative of the anterior and posterior wall sections of the bladder 41 delimiting this bladder section. The positioning of the trajectory of the ultrasound beams 30' onto this bladder model may be based on the determined orientation angle θ of the wearable bladder monitoring device 1, such that a distinction can be made between trajectories resulting from different orientation angles of the wearable bladder monitoring device 1, such as example orientation angles θ₁ and θ₂ as schematically depicted in FIG. 7.

Based on the trajectories fitted onto the bladder model and the respective diameters (cross-sectional lengths) of the bladder 41 along these respective trajectories, the signal processor 23 may in operation 117 estimate a bladder volume of the bladder 41. This for example may be based on the number of ultrasound beams 30' and the respective beam angles of these beams as well as on a defined orientation of the bladder model relative to the pelvic bone 43, such that a portion of the bladder model obscured from the ultrasound beams 30 by the pelvic bone 43 as a function of the orientation angle θ of the wearable bladder monitoring device 1 can be estimated from the determined orientation angle θ. Consequently, the volume of the portion of the bladder 41 visible to the ultrasound beams 30' may be extrapolated to a total bladder volume in this manner.

In an embodiment, the wearable bladder monitoring device 1 may be adapted to periodically repeat the monitoring of the bladder at a defined monitoring frequency such as to monitor changes in the bladder volume over the monitoring period. This for example may be used to determine a rate of change in the bladder volume, from which diagnostic observations may be derived, such as a rehydration or dehydration rate of the subject 40. Also, monitoring the bladder volume over a period of time may provide valuable insights into the retention volume of the bladder 41, which has previously explained is an indicator of prostate and urinary tract conditions, with the consequent bladder stretching being a key contributor to the occurrence of delirium in elderly subjects 40. In order to preserve the battery life of the battery 15, the defined monitoring frequency should be chosen as low as possible. In an embodiment, the signal processor 23 is adapted to define the monitoring frequency from an evaluation of a previous bladder monitoring event or set of bladder monitoring events, e.g. based on a previously determined filling or emptying rate of the bladder 41 or on a suspected pathology of the bladder 41.

The method 100 optionally may further comprise the generation of a warning signal in operation 119 with the signal processor 23, which warning signal for example may be generated as an audible or visible warning signal with the remote device 5 under control of the signal processor 23. The signal processor 23 may generate the warning signal in response to the estimation of a particular bladder volume or change in the bladder volume. Such a warning signal for example may be generated to wake a sleeping subject 40 in case of the bladder 41 of the subject 40 gets overly full, e.g. in order to prevent wetting of the subject's bed in case of incontinence. Alternatively, such a warning signal may be used to inform an athlete about his or her hydration levels, e.g. to ensure appropriate post-exercise rehydration. Alternatively, the warning signal may be generated by an alarming system connected to the remote data repository, which alarming system may be adapted to process the data uploaded into the remote data repository by the wearable bladder monitoring device 1 and to generate the warning signal based on said processing. The method 100 may subsequently terminate in operation 121.

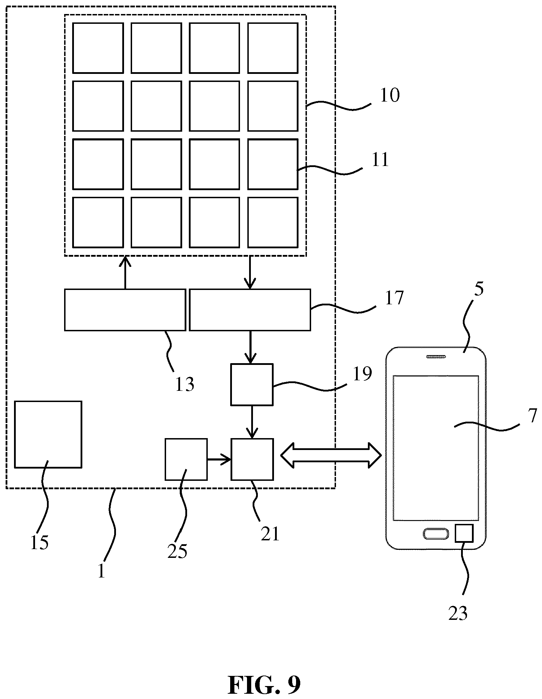

In order to further refine the bladder volume estimation from the data pertaining to the echo signals generated with the wearable bladder monitoring device 1, this device may further comprise an orientation sensor 25 as schematically depicted in FIG. 9. Such an orientation sensor 25, e.g. an accelerometer or the like, is typically adapted to determine the orientation, e.g. posture or pose, of the subject 40, as different orientations of the subject 40 may lead to changes in the shape of the bladder 41. Therefore, by including the orientation data provided by the orientation sensor 25 in the data to be provided to the signal processor 23, the signal processor 23 may estimate the bladder volume of the bladder 41 of the subject 40 based on the combination of the data pertaining to the echo signals 31 of the ultrasound beams 30 and the orientation data provided by the orientation sensor 25.

In an embodiment, the signal processor 23 has access to a plurality of bladder models, e.g. stored in a data storage device (not shown), which may be contained by the device also containing the signal processor 23, e.g. the remote device 5 or the wearable bladder monitoring device 1, or may be a remote data storage device accessible over a network such as the Internet by the signal processor 23, e.g. through wireless communication module as will be readily understood by the skilled person. Each bladder model may be associated with a particular orientation (posture or pose) of the subject 40, such that the appropriate bladder model may be selected by the signal processor 23 based on the orientation data generated with the orientation sensor 25.

A further refinement of the accuracy of the bladder volume estimation may be obtained by optimizing the resonance frequencies of the ultrasound beams 30' passing through the bladder 41. This can be understood as follows. As previously explained, the echo signals 31' of the ultrasound beams 30' exhibit a pair of 'steep' intensity changes 33 and 33' corresponding to the ultrasound beam 30' passing through an anterior wall section and a posterior wall section respectively of the bladder 41. In order to accurately locate the respective positions of these anterior and posterior wall sections, this intensity change preferably should be as steep as possible, such that the distance over which this intensity change is spread is minimized, thereby minimizing the uncertainty in the exact locations of these wall sections and consequently improving the accuracy of the estimated distance, i.e. bladder diameter or cross-sectional length, between these wall sections.

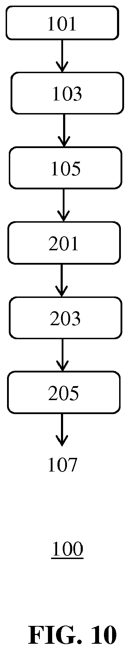

This may be achieved by tuning the frequency of the ultrasound beams 30' as a function of the actual depth and size of the bladder 41, as will be explained in further detail with the aid of FIG. 10, which depicts a flowchart of a refinement of the method 100 in which such frequency optimization is deployed. As is well-known per se, the ultrasound frequency is proportional to the image resolution and inversely proportional to the imaging depth. Hence, for a bladder 41 there exists an optimal ultrasound frequency f1 for the measurement of the anterior wall section of the bladder 41 as well as an optimal ultrasound frequency f2 for the measurement of the posterior wall section of the bladder 41, with typically f1 ≠ f2 due to the different distances of the anterior and posterior wall sections from the abdominal surface of the subject 40.

Hence, in an embodiment the wearable bladder monitoring device 1 is adapted to tune the ultrasound frequency of the ultrasound beams 30', i.e. at least of the ultrasound beams passing through the bladder 41, in order to improve the accuracy of the anterior and posterior wall section localizations. To this end, in operation 103 the phased array controller 13 may control the phased array 10 to generate ultrasound beams at various beam angles at an initial frequency fᵢ, which initial frequency typically is a relatively low frequency, e.g. a frequency of about 2MHz, in order to generate a relatively wide angle ultrasound beam, with the echo signals induced by these ultrasound beams being acquired in operation 105 as previously explained. This enables the bladder monitoring system to roughly determine the position and size of the bladder 41 due to the wide angled nature of the low frequency ultrasound beam.

This rough estimation of the position and size of the bladder 41 is used in operation 201 to determine a first distance dₐ to the anterior wall of the bladder 41 and a second distance d_{b} to the posterior wall of the bladder 41, which distances in combination with the attenuation rate of ultrasound waves in fat tissue, e.g. about 0.5dB/MHz/cm, to determine an optimal ultrasound frequencies f1 and f2 for imaging the anterior and posterior walls of the bladder 41, e.g. using the formula f = M/(0.5^{∗}D), in which f is the optimal ultrasound frequency, M is the maximum attenuation threshold for the signal to noise ratio specific phased array 10 and D is the distance to the wall section to be imaged.

Subsequently, the bladder 41 may be imaged in operation 203 with ultrasound signals at the determined optimal ultrasound frequencies f1 and f2 with the respective echoes of these ultrasound signals being acquired in operation 205 such that the bladder volume estimation with the signal processor 23 may be based on the echo signals of the ultrasound beams at the optimal ultrasound frequencies f1 and f2 in order to improve the accuracy of the bladder volume estimation. This for example may be achieved by combining the ultrasound beams at the optimized frequencies under various beam angles as previously explained into a single evaluation, e.g. by mapping the echo signals of these ultrasound beams onto a bladder model as previously explained. Alternatively, rather than performing multiple ultrasound measurements at the respective optimal ultrasound frequencies f1 and f2, a single measurement (scan) may be performed at an ultrasound frequency that is an average of the optimal ultrasound frequencies f1 and f2, e.g. a weighted average at 110% of the optimal ultrasound frequency f2 for imaging the posterior wall sections, as f2 typically is lower than f1.

As previously explained, the acquired echo signals may be pre-processed in operation 107 and further processed as explained in more detail with the aid of the flowchart of FIG. 8. It is noted that where such frequency optimization of the ultrasound beams is to be included in the operation of the wearable bladder monitoring device 1, the ultrasound transducer elements 11 preferably are CMUT elements due to their superior bandwidth characteristics compared to e.g. PZT elements.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A bladder monitoring system comprising a signal processor (23) adapted to:
receive data from a wearable bladder monitoring device (1), said device including:
securing means (27, 29) for securing the device to a subject's (40) body;
a phased array (11) of ultrasound transducers (10); and
a phased array controller (13) adapted to control the phased array to direct a plurality of ultrasound beams (30, 30', 30") into the subject's body under a range of beam angles, wherein said data pertains to echo signals (31, 31', 31") of said ultrasound beams; and
wherein the signal processor is adapted to receive said data from the phased array and to process said data in order to;
identify an edge of the subject's pelvic bone (43) proximal to the subject's bladder (41) from data pertaining to at least one of said echo signals;
determine an orientation of the wearable bladder monitoring device relative to the pelvic bone based on beam angle information associated with the at least one of said echo signals; and
derive bladder information from the data based on the trajectories of the ultrasound beams passing through the bladder as a function of the determined orientation of the wearable bladder monitoring device relative to the pelvic bone.

2. The bladder monitoring system of claim 1, further comprising the wearable bladder monitoring device (1).

3. The bladder monitoring system of claim 2, wherein the wearable bladder monitoring device (1) further comprises the signal processor (23).

4. The bladder monitoring system of claim 2 or 3, wherein the wearable bladder monitoring device (1) further comprises an orientation sensor (25) for determining an orientation of the subject (40), and wherein the signal processor (23) is adapted to determine an orientation of the wearable bladder monitoring device relative to the pelvic bone (43) based on beam angle information associated with the at least one of said echo signals (31") and an orientation signal from the orientation sensor.

5. The bladder monitoring system of claim 4, further comprising a data storage device storing a plurality of bladder models, each associated with a particular orientation of the subject (40), wherein the signal processor (23) is adapted to retrieve the defined bladder model from the data storage device based on said orientation signal.

6. The bladder monitoring system of any of claims 1-5, wherein the signal processor (23) is adapted to estimate a diameter of the subject's bladder (40) for each echo signal (31') from the data pertaining to a subset of said echo signals.

7. The bladder monitoring system of claim 6, wherein the signal processor (23) is further adapted to, for each echo signal in said subset:
calculate a first optimal frequency (f1) of its associated ultrasound beam (30') for imaging an anterior boundary (41a) of the bladder (41) at which a slope of an intensity change of said associated ultrasound beam when passing through said anterior boundary is maximized;
calculate a second optimal frequency (f2) of its associated ultrasound beam for imaging a posterior boundary (41b, 41c) of the bladder at which a slope of an intensity change of said associated ultrasound beam when passing through said posterior boundary is maximized;
instruct the phased array controller (13) to generate at least one further ultrasound beam under the beam angle of the associated ultrasound beam with the phased array (10), said at least at least one further ultrasound beam having a frequency corresponding to at least one of the first optimal frequency and the second optimal frequency or an average of the first optimal frequency and the second optimal frequency; and
estimate the diameter of the subject's bladder for said beam angle from the at least one further echo signal of the at least one further ultrasound beam.

8. The bladder monitoring system of claim 7, wherein the at least one further ultrasound beam comprises:
a first further ultrasound beam having the first optimal frequency (f1) and a second further ultrasound beam having the second optimal frequency (f2).

9. The bladder monitoring system of any of claims 6-8, wherein the signal processor (23) is adapted to estimate a bladder volume by fitting the estimated diameters (d1, d2) of the subject's bladder (41) to a defined bladder model and estimating the bladder volume from the fitting result.

10. The bladder monitoring system of any of claims 1-9, wherein the phased array controller (13) is adapted to periodically generate said plurality of ultrasound beams (30, 30', 30").

11. The bladder monitoring system of claim 10, wherein the signal processor (23) is adapted to derive a bladder function from the data pertaining to the respective echo signals (31') of the periodically generated pluralities of ultrasound beams (30').

12. The bladder monitoring system of claim 10 or 11, wherein a frequency of said periodic generation is based on a previously determined bladder function by the signal processor (23).

13. The bladder monitoring system of any of claims 1-12, wherein the securing means include a strap (29) attached to the wearable bladder monitoring device (1) or an adhesive layer (27) on a subject-facing surface of the wearable bladder monitoring device.

14. The bladder monitoring system of any of claims 1-13, wherein the wearable bladder monitoring device (1) further comprises a wireless communication module (21) for communicating with a remote device (5).

15. A method (100) of monitoring a bladder with the bladder monitoring system of any of claims 1-14, the method comprising:
securing the wearable bladder monitoring device (1) to a subject's body;
generating a plurality of ultrasound beams (30, 30', 30") under a range of beam angles with the phased array (10);
receiving echo signals (31, 31', 31") of said ultrasound beams from the phased array; and processing data pertaining to said echo signals in order to:
identify an edge of the subject's pelvic bone (43) proximal to the subject's bladder (41) from data pertaining to at least one of said echo signals;
determine an orientation of the wearable bladder monitoring device relative to the pelvic bone based on beam angle information associated with the at least one of said echo signals; and
derive bladder information from the data based on the trajectories of the ultrasound beams passing through the bladder as a function of the determined orientation of the wearable bladder monitoring device relative to the pelvic bone.

## Patentansprüche

1. Ein Blasenüberwachungssystem, das einen Signalprozessor (23) umfasst, der folgende Schritte durchführt:
Abrufen der Daten von einem tragbaren Blasenüberwachungsgerät (1), wobei dieses Gerät Folgendes umfasst:
Befestigungsvorrichtungen (27, 29) zum Befestigen des Geräts am Körper des Patienten (40);
Phased-Array-(11)-Ultraschallwandler (10); und
eine Phased-Array-Steuerung (13) zum Steuern des Phased Array, um Ultraschallstrahlen (30, 30',30") in einer Reihe von Strahlungswinkeln in den Körper des Patienten zu leiten, wobei die Daten zu den Echosignalen (31, 31', 31") der Ultraschallstrahlen gehören; und
wobei der Signalprozessor die Daten vom Phased Array abruft und verarbeitet, um;
Ermitteln eines proximal zur Blase (41) des Patienten verlaufenden Rands des Beckenknochens (43) anhand von Daten, die zu mindestens einem der Echosignale gehören;
Ermitteln der Ausrichtung des tragbaren Blasenüberwachungsgeräts relativ zum Beckenknochen beruhend auf den dem mindestens einen Echosignal zugeordneten Strahlenwinkeldaten; und
Abrufen der Blasendaten beruhend auf dem Ultraschallstrahlverlauf durch die Blase als Funktion der ermittelten Ausrichtung des tragbaren Blasenüberwachungsgeräts relativ zum Beckenknochen.

2. Das Blasenüberwachungssystem gemäß Anspruch 1,
das zudem das tragbare Blasenüberwachungsgerät (1) umfasst.

3. Das Blasenüberwachungssystem gemäß Anspruch 2,
wobei das tragbare Blasenüberwachungsgerät (1) zudem den Signalprozessor (23) umfasst.

4. Das Blasenüberwachungssystem gemäß Anspruch 2 oder 3,
wobei das tragbare Blasenüberwachungsgerät (1) zudem einen Ausrichtungssensor (25) zum Ermitteln der Ausrichtung des Patienten (40) umfasst, und wobei der Signalprozessor (23) die Ausrichtung des tragbaren Blasenüberwachungsgeräts relativ zum Beckenknochen (43) beruhend auf den dem mindestens einen Echosignal (31") sowie dem Ausrichtungssignal des Ausrichtungssensors zugeordneten Strahlenwinkeldaten ermittelt.

5. Das Blasenüberwachungssystem gemäß Anspruch 4,
das zudem über ein Datenspeichergerät verfügt, auf dem mehrere Blasenmodelle gespeichert sind, die jeweils einer bestimmten Ausrichtung des Patienten (40) zugeordnet sind, wobei der Signalprozessor (23) anhand des Ausrichtungssignals das definierte Blasenmodell vom Datenspeichergerät abruft.

6. Das Blasenüberwachungssystem gemäß einer der Ansprüche 1 bis 5,
wobei der Signalprozessor (23) anhand der zu einer Untermenge der Echosignale gehörenden Daten für die einzelnen Echosignale (31') den Durchmesser der Blase des Patienten (40) schätzt.

7. Das Blasenüberwachungssystem gemäß Anspruch 6,
wobei der Signalprozessor (23) zudem für die einzelnen Echosignale der Untermenge folgende Schritte durchführt:
Berechnen einer ersten optimalen Frequenz (f1) des zugeordneten Ultraschallstrahls (30') für das Abbilden einer vorderen Grenze (41a) der Blase (41), an der der Anstieg der Intensitätsveränderung des zugeordneten Ulraschallstrahls beim Durchlaufen der vorderen Grenze maximiert wird;
Berechnen einer zweiten optimalen Frequenz (f2) des zugeordneten Ultraschallstrahls für das Abbilden einer hinteren Grenze (41b, 41c) der Blase, an der der Anstieg der Intensitätsveränderung des zugeordneten Ulraschallstrahls beim Durchlaufen der hinteren Grenze maximiert wird;
die Phased-Array-Steuerung (13) anweisen, mit dem Phased Array (10) mindestens einen weiteren Ultraschallstrahl mit dem Strahlungswinkel des zugeordneten Ultraschallstrahls zu generieren, wobei der mindestens eine zusätzliche Ultraschallstrahl über eine Frequenz verfügt, die mindestens der ersten und der zweiten optimalen Frequenz oder einem Durchschnittswert der ersten und der zweiten optimalen Frequenz entspricht; und
Schätzen des Durchmessers der Blase des Patienten für den besagten Strahlungswinkel anhand des mindestens einen zusätzlichen Echosignals des mindestens einen zusätzlichen Ultraschallstrahls.

8. Das Blasenüberwachungssystem gemäß Anspruch 7,
wobei der mindestens eine zusätzliche Ultraschallstrahl Folgendes umfasst:
einen ersten zusätzlichen Ultraschallstrahl mit der ersten optimalen Frequenz (f1) sowie einen zweiten zusätzlichen Ultraschallstrahl mit der zweiten optimalen Frequenz (f2).

9. Das Blasenüberwachungssystem gemäß einer der Ansprüche 6 bis 8,
wobei der Signalprozessor (23) ein Blasenvolumen schätzt, indem er die geschätzten Durchmesser (d1, d2) der Blase des Patienten (41) in ein definiertes Blasenmodell einpasst und das Blasenvolumen anhand des Einpassungsergebnisses schätzt.

10. Das Blasenüberwachungssystem gemäß einer der Ansprüche 1 bis 9,
wobei die Phased-Array-Steuerung (13) regelmäßig die Ultraschallstrahlen (30, 30', 30") erzeugt.

11. Das Blasenüberwachungssystem gemäß Anspruch 10,
wobei der Signalprozessor (23) von den zu den entsprechenden Echosignalen (31') der regelmäßig erzeugten Ultraschallstrahlen (30') gehörenden Daten eine Blasenfunktion ableitet.

12. Das Blasenüberwachungssystem gemäß Anspruch 10 oder 11, wobei die Frequenz für das regelmäßige Erzeugen auf der zuvor vom Signalprozessor (23) ermittelten Blasenfunktion beruht.

13. Das Blasenüberwachungssystem gemäß einer der Ansprüche 1 bis 12,
wobei die Befestigungsvorrichtung eine Schlaufe (29) am tragbaren Blasenüberwachungsgerät (1) oder eine Klebeschicht (27) auf einer dem Patienten zugewandten Fläche des tragbaren Blasenüberwachungsgeräts umfasst.

14. Das Blasenüberwachungssystem gemäß einer der Ansprüche 1 bis 13,
wobei das tragbare Blasenüberwachungsgerät (1) zudem ein Drahtloskommunikationsmodul (21) für die Kommunikation mit einem Remote-Gerät (5) umfasst.

15. Eine Methode (100) zum Überwachen einer Blase mit dem Blasenüberwachungssystem gemäß einer der Ansprüche 1 bis 14, wobei die Methode folgende Schritte umfasst:
Befestigen des tragbaren Blasenüberwachungsgeräts (1) am Körper des Patienten;
Generieren mehrerer Ultraschallstrahlen (30, 30', 30") in einer Reihe von Strahlungswinkeln mit dem Phased Array (10);
Abrufen der Echosignale (31, 31', 31") der Ultraschallstrahlen vom Phased Array;
und Verarbeiten der zu den Echosignalen gehörenden Daten, um folgende Schritte durchzuführen:
Ermitteln eines proximal zur Blase (41) des Patienten verlaufenden Rands des Beckenknochens (43) anhand von Daten, die zu mindestens einem der Echosignale gehören;
Ermitteln der Ausrichtung des tragbaren Blasenüberwachungsgeräts relativ zum Beckenknochen beruhend auf den dem mindestens einen Echosignal zugeordneten Strahlenwinkeldaten; und
Abrufen der Blasendaten beruhend auf dem Ultraschallstrahlverlauf durch die Blase als Funktion der ermittelten Ausrichtung des tragbaren Blasenüberwachungsgeräts relativ zum Beckenknochen.

## Revendications

1. Système de surveillance de la vessie comprenant un processeur de signal (23) conçu : pour recevoir des données d'un dispositif de surveillance de la vessie (1) portable, ledit dispositif comprenant :
des moyens de fixation (27, 29) pour fixer le dispositif au corps d'un sujet (40) ;
un réseau à commande de phase (11) de transducteurs ultrasonores (10) ; et
un dispositif de commande de réseau à commande de phase (13) conçu pour commander le réseau à commande de phase pour diriger une pluralité de faisceaux ultrasonores (30, 30', 30") dans le corps du sujet sous une plage d'angles de faisceau, dans lequel lesdites données se rapportent à des signaux d'écho (31, 31', 31") desdits faisceaux ultrasonores ; et dans lequel le processeur de signal est conçu pour recevoir lesdites données du réseau à commande de phase et pour traiter lesdites données ;
pour identifier un bord de l'os pelvien du sujet (43) à proximité de la vessie du sujet (41) en fonction des données se rapportant à au moins l'un desdits signaux d'écho ;
pour déterminer une orientation du dispositif de surveillance de la vessie portable par rapport à l'os pelvien en fonction des informations d'angle de faisceau associées à l'au moins un desdits signaux d'écho ; et
pour dériver des informations de vessie en fonction des données en fonction des trajectoires des faisceaux ultrasonores traversant la vessie en fonction de l'orientation déterminée du dispositif de surveillance de la vessie portable par rapport à l'os pelvien.

2. Système de surveillance de la vessie selon la revendication 1, comprenant en outre le dispositif de surveillance de la vessie (1) portable.

3. Système de surveillance de la vessie selon la revendication 2, dans lequel le dispositif de surveillance de la vessie (1) portable comprend en outre le processeur de signal (23).

4. Système de surveillance de la vessie selon la revendication 2 ou 3, dans lequel le dispositif de surveillance de la vessie (1) portable comprend en outre un capteur d'orientation (25) pour déterminer une orientation du sujet (40), et dans lequel le processeur de signal (23) est conçu pour déterminer une orientation du dispositif de surveillance de la vessie portable par rapport à l'os pelvien (43) en fonction des informations d'angle de faisceau associées à l'au moins un desdits signaux d'écho (31") et d'un signal d'orientation du capteur d'orientation.

5. Système de surveillance de la vessie selon la revendication 4, comprenant en outre un dispositif de mémorisation de données mémorisant une pluralité de modèles de vessie, chacun associé à une orientation particulière du sujet (40), dans lequel le processeur de signal (23) est conçu pour récupérer le modèle de vessie défini en fonction du dispositif de mémorisation de données en fonction dudit signal d'orientation.

6. Système de surveillance de la vessie selon l'une quelconque des revendications 1 à 5, dans lequel le processeur de signaux (23) est conçu pour estimer un diamètre de la vessie du sujet (40) pour chaque signal d'écho (31') en fonction des données se rapportant à un sous-ensemble desdits signaux d'écho.

7. Système de surveillance de la vessie selon la revendication 6, dans lequel le processeur de signal (23) est en outre conçu, pour chaque signal d'écho dans ledit sous-ensemble :
pour calculer une première fréquence optimale (f1) de son faisceau ultrasonore (30') associé pour imager une limite antérieure (41a) de la vessie (41) à laquelle une pente d'un changement d'intensité dudit faisceau ultrasonore associé lors du passage à travers ladite limite antérieure est maximisée ;
pour calculer une seconde fréquence optimale (f2) de son faisceau ultrasonore associé pour imager une limite postérieure (41b, 41c) de la vessie à laquelle une pente d'un changement d'intensité dudit faisceau ultrasonore associé lors du passage à travers ladite limite postérieure est maximisée ;
pour ordonner au dispositif de commande de réseau à commande de phase (13) de générer au moins un autre faisceau ultrasonore sous l'angle de faisceau du faisceau ultrasonore associé avec le réseau à commande de phase (10), ledit au moins un autre faisceau ultrasonore présentant une fréquence correspondant à la première fréquence optimale et/ou de la seconde fréquence optimale et/ou une moyenne de la première fréquence optimale et de la seconde fréquence optimale ; et
pour estimer le diamètre de la vessie du sujet pour ledit angle de faisceau en fonction de l'au moins un autre signal d'écho de l'au moins un autre faisceau ultrasonore.

8. Système de surveillance de la vessie selon la revendication 7, dans lequel l'au moins un autre faisceau ultrasonore comprend :
un premier autre faisceau ultrasonore présentant la première fréquence optimale (f1) et un second autre faisceau ultrasonore présentant la seconde fréquence optimale (f2).

9. Système de surveillance de la vessie selon l'une quelconque des revendications 6 à 8, dans lequel le processeur de signal (23) est conçu pour estimer un volume de vessie par ajustement des diamètres estimés (d1, d2) de la vessie du sujet (41) à un modèle de vessie défini et par estimation du volume de la vessie en fonction du résultat de l'ajustement.

10. Système de surveillance de la vessie selon l'une quelconque des revendications 1 à 9, dans lequel le dispositif de commande de réseau à commande de phase (13) est conçu pour générer périodiquement ladite pluralité de faisceaux ultrasonores (30, 30', 30").

11. Système de surveillance de la vessie selon la revendication 10, dans lequel le processeur de signaux (23) est conçu pour dériver une fonction de vessie en fonction des données relatives aux signaux d'écho (31') respectifs des pluralités de faisceaux ultrasonores (30') générées périodiquement.

12. Système de surveillance de la vessie selon la revendication 10 ou 11, dans lequel une fréquence de ladite génération périodique est basée sur une fonction de vessie préalablement déterminée par le processeur de signal (23).

13. Système de surveillance de la vessie selon l'une quelconque des revendications 1 à 12, dans lequel les moyens de fixation comprennent une sangle (29) attachée au dispositif de surveillance de la vessie (1) portable ou une couche adhésive (27) sur une surface faisant face au sujet du dispositif de surveillance de la vessie portable.

14. Système de surveillance de la vessie selon l'une quelconque des revendications 1 à 13, dans lequel le dispositif de surveillance de la vessie (1) portable comprend en outre un module de communication sans fil (21) pour communiquer avec un dispositif distant (5).

15. Procédé (100) de surveillance d'une vessie comportant le système de surveillance de la vessie selon l'une quelconque des revendications 1 à 14, ledit procédé comprenant :
la fixation du dispositif de surveillance de la vessie (1) portable au corps d'un sujet ;
la génération d'une pluralité de faisceaux ultrasonores (30, 30', 30") sous une plage d'angles de faisceau avec le réseau à commande de phase (10) ;
la réception des signaux d'écho (31, 31', 31") desdits faisceaux ultrasonores en fonction du réseau à commande de phase ; et le traitement des données relatives auxdits signaux d'écho ;
pour identifier un bord de l'os pelvien du sujet (43) à proximité de la vessie du sujet (41) en fonction des données se rapportant à au moins l'un desdits signaux d'écho ;
pour déterminer une orientation du dispositif de surveillance de la vessie portable par rapport à l'os pelvien en fonction des informations d'angle de faisceau associées à l'au moins un desdits signaux d'écho ; et
pour dériver des informations de vessie en fonction des données en fonction des trajectoires des faisceaux ultrasonores traversant la vessie en fonction de l'orientation déterminée du dispositif de surveillance de la vessie portable par rapport à l'os pelvien.
